# NEUE EUROPÄISCHE PATENTSCHRIFT

(11) **EP 1 062 923 B2**
(45) Veröffentlichungstag und Bekanntmachung des Hinweises auf die Entscheidung über den Einspruch: **19.04.2006**
(45) Hinweis auf die Patenterteilung: 14.01.2004
(21) Anmeldenummer: 00112385.0
(22) Anmeldetag: 09.06.2000
(51) Int. Cl.: A61F 2/36

(54) **Hüftgelenk-Endoprothesensystem**
Hip joint endoprostheses system
Système d'endoprothèses pour l'articulation de la hanche

(30) Priorität: 23.06.1999 DE 19928709
(43) Veröffentlichungstag der Anmeldung: 27.12.2000
(73) Patentinhaber: Plus Orthopedics AG, 6343 Rotkreuz (CH)
(72) Erfinder: Zweymüller, Karl, Prof. Dr. med., 1190 Wien (AT)
(74) Vertreter: Popp, Eugen

(56) Entgegenhaltungen:
- EP-A- 0 032 165
- EP-A- 0 144 209
- EP-A- 0 477 113
- DE-T2- 69 116 053
- DE-U- 8 901 018
- FR-A- 2 429 010
- FR-A- 2 574 283
- FR-A- 2 610 824
- FR-A- 2 631 543
- FR-A- 2 636 837
- FR-A- 2 639 820
- FR-A- 2 667 785
- FR-A- 2 701 835
- FR-A- 2 753 081
- GB-A- 2 281 213
- US-A- 4 068 324
- US-A- 5 108 451
- Publikation, R. Davey und E. Tozakoglou, A scientific exhibit at the 1999 AAOS Meeting, Anaheim, Kalifornien USA "The Role of Lateral Offset Stems"
- Prospekt der Fa. Protek Ltd., 3012 Bern, Schweiz, 1981, "Original M.E. Mueller Straight Stem Total Hip Replacement System"
- Prospekt der Fa. Protek Ltd., 3001 Bern, Schweiz, 1982, "Original M.E. Mueller Straight Stem Total Hip Replacement System"
- Prospekt der Fa. Protek Ltd., 3001 Bern, Schweiz, 1983, "Original M.E. Mueller Straight Stem Total Hip Replacement System"
- "Die Geradschaftsprothese nach M.E.Mueller; Konsequenz eines biomechanischen Prinzips"; Sonderdruck aus: Robert Schneider, "Die Totalprothese der Hüfte; Ein biomechanisches Konzept und seine Konsequenzen", Verlag Hans Huber, Bern, Stuttgart, Toronto, 1987, pp. 233-236

## Beschreibung

Die Erfindung betrifft ein Hüftgelenk-Endoprothesensystem nach dem Oberbegriff des Anspruches 1.

Hüftgelenk-Endoprothesen mit einem blattartigen Schaft sind aus der EP 0 032 165 B1 bekannt.

Bei dieser bekannten Konstruktion erweitert sich der Schaft vom distalen Ende aus in Richtung seiner Längsachse bis in einen Bereich zwischen etwa 2/3 und 3/4 der Schaftlänge - gemessen entlang der Längsachse - etwa allseitig konisch. Die mediale Schmalseite des Schaftes geht aus dem Konus heraus in einen stetig gekrümmten Bogen über, der an einem kragenartigen Ansatz endet. Dieser Ansatz trennt einen Femur-Verankerungsabschnitt des Schaftes von einem Prothesenhals, der einen sich nach proximal konisch verjüngenden Zapfen umfaßt, welcher zur Aufnahme eines kugelförmigen Gelenkkopfes dient. Die Prothesenhalsachse schneidet die Längsachse des Schaftes unter einem Winkel, der im wesentlichen dem Winkel zwischen dem Schenkelhals und der Femurachse eines natürlichen Hüftgelenkes entspricht.

Die bekannte Konstruktion hat sich bewährt, um eine sogenannte "Schlußrotation" beim Einsetzen der Prothese zu vermeiden, ohne die Resektionsebene am Schenkelhals zu tief ansetzen zu müssen. Diese nachteilige "Schlußrotation" besteht darin, daß es beim vollständigen Einsetzen der konventionellen Prothesenschäfte wegen der erforderlichen Mindestdicke des Schaftblattes häufig zu einer Rotation des Schaftes im Femurknochen kommt, da infolge der mehrfachen Krümmungen des proximalen Femurendes ein gerader oder auch leicht gebogener Gegenstand von der Wand des Oberschenkelknochens abgelenkt wird.

In der klinischen Praxis hat sich gezeigt, daß für große Patientengruppen, die ein künstliches Hüftgelenk benötigen, zwar jeweils die Gesamtlänge - verstanden als Projektion der Gesamterstreckung des Prothesenschaftes von dessen distalem Ende bis zum äußersten Ende des am Prothesenhals angeformten Zapfens - im wesentlichen gleich ist, jedoch aufgrund der individuellen anatomischen Besonderheiten gleichwohl Prothesenschäfte mit unterschiedlichen Abmessungen benötigt werden, deren Einsatz auch unterschiedliche Werkzeuge zur Präparation des Femurs erfordert. Genauere Untersuchungen haben ergeben, daß innerhalb solcher Patientengruppen die anatomischen Unterschiede hauptsächlich in einem Unterschied des Abstandes des Hüftgeienkkugel-Drehpunktes zur Femurlängsachse bestehen.

Die Veröffentlichung FR-A-2 429 010 beschreibt ein Hüftgelenk-Endoprothesensystem, bei dem verschiedene Abstände zwischen der Schaft-Längsachse und dem Mittelpunkt der Gelenkkugel dadurch erreicht werden, daß der Prothesenhats in zwei verschiedenen Längen zur Verfügung gestellt wird, und daß die konische Bohrung in der Gelenkkugel in zwei verschiedenen Größen bzw. Tiefen zur Verfügung gestellt wird. Durch Kombination lassen sich vier verschiedene Abstände realisieren (Seite 1, Zeilen 1-18). Durch den gleichbleibenden Winkel zwischen Schaftlängs-achse und Prothesenhals ändert sich hier jedoch gleichzeitig die auf die Schaftlängsachse projizierte Gesamtlänge der Prothese und der Oberschenkelknochen muß je nach gewählter Prothese unterschiedlich ausgefräst werden.
Durch die Publikation, R. Davey und E. Tozakoglou, A Scientific Exhibit At The 1999 AAOS Meeting, Anaheim, Kalifornien, USA, "The Role Of Lateral Offset Stems", ist es bekannt, eine Konstanz der Gesamtlänge des Prothesenschaftes bei unterschiedlich langen Prothesenhälsen dadurch zu erreichen, daß der zwischen der Prothesenhalsachse und der Längsachse des Verankerungsabschnittes eingeschlossene Winkel in Abhängigkeit von der Länge des Prothesenhalses unterschiedlich gewählt wird. Der unterschiedliche Offset des Schaftes wird durch Verkippen des Prothesenhalses erreicht, und zwar derart, daß die auf die Schaftlängsachse projizierte Gesamtlänge der Prothese konstant bleibt. Nachteilig ist jedoch, daß nach dem Kippen der Prothesenhalsachse sich der Schnittpunkt mit der Schaftlängsachse verändert, wodurch der Nullpunkt des lokalen Koordinaten-systems für eine präoperative Planung verlorengeht. Dementsprechend müßte der Arzt beim Einsatz des bekannten Systems bei einem intra-operativen Wechsel des Schafttyps eine Anpassung am Koordinatennullpunkt vornehmen. Diese Fehlerquelle gilt es zu vermeiden.
Ähnlich verhält es sich im übrigen bei der sogenannten "Müller-Geradschaftprothese". Es wird diesbezüglich auf die Prospekte der Firma Protek Ltd., 3001 Bern, Schweiz, aus den Jahren 1981, 1982 und 1983 jeweils mit dem Titel "Original M.E. Müller Straight Stem Total Hip Replacernent System" verwiesen, wo vorgeschlagen ist, die Prothesenhalsachse in toto parallel zu verschieben. Dadurch ändert sich zwangsläufig auch der Schnittpunkt zwischen Prothesenhalsachse und Schaftlängsachse.

Auf den oben genannten Erkenntnissen beruht die nachfolgend genannte Aufgabenstellung.

Der Erfindung liegt die Aufgabe zugrunde, ein gattungsgemäßes Hüftgelenk-Endoprothesensystem anzugeben, das eine Zeit- und Kostenersparnis beim Einsatz, insbesondere bei den Vorbereitungsarbalten für die Implantation des Prothesenschaftes, erbringt, und bei dem bei einem intra-operativem Wechsel des Schafttyps keine Anpassung am lokalen Koordinaten-Nullpunkt vorgenommen werden muß.

Diese Aufgabe wird durch ein Hüftgelenk-Endoprothesensystem mit den Merkmalen des Anspruchs 1 gelöst.

Die Erfindung schließt den wesentlichen Gedanken ein, ein Hüftgelenk-Endoprothesensystem mit - bezogen auf die Längsachse des Prothesenschaftes - im wesentlichen konstanter Länge anzugeben. Dieses System hat den wesentlichen Vorteil, daß bei der Vorbereitung des Femurs für die Implantation mit einem einheitlichen Raspelmaß für alle Prothesengrößen des Systems gearbeitet werden kann.

Weiter schließt die Erfindung den Gedanken ein, diese Konstanz der Gesamtlänge des Prothesenschaftes bei unterschiedlich langen Prothesenhälsen in einer Variante dadurch zu erreichen, daß der zwischen der Prothesenhalsachse und der Längsachse des Verankerungsabschnittes eingeschlossene Winkel in Abhängigkeit von der Länge des Prothesenhalses unterschiedlich gewählt wird. In einer anderen Variante wird dieser Winkel (der sogenannte "CCD-Winkel") konstant gehalten, jedoch in den Längsverlauf des Prothesenhalses ein Versatz-Abschnitt ("Offset") eingefügt, wobei der Betrag des vorzusehenden seitlichen Versatzes in Abhängigkeit von der benötigten Prothesenhalslängerespektive in Abhängigkeit vom zu realisierenden Abstand zwischen der Längsachse des Verankerungsabschnittes und demjenigen Punkt ("Referenzpunkt") im Bereich des Prothesenhalszapfens bestimmt wird, der den Mittelpunkt bzw. Drehpunkt der später aufgesetzten Gelenkkugel angibt. Grundsätzlich ist auch eine Kombination beider Varianten möglich.

Die zweite Variante ist eher bei größeren Prothesenhalslängen sinnvoll, für die sich zur Gewährleistung der einheitlichen Gesamtlänge der Prothesenschäfte innerhalb des Systems ein unvorteilhaft kleiner Prothesenhalswinkel ergeben könnte. In solchen Fällen wiegen die mit dem Prothesenhals-Offsetabschnitt erreichbaren Vorteile die möglichen Nachteile der mit dem Einführen des Offset verbundenen Irregularität (eines "Knickes") in der medialen Bogenlinie (die unter Umständen zu hoch belasteten Druckstellen im entsprechenden Bereich der Spongiosa führen kann) wieder auf. Im Bereich nicht allzu langer Prothesenhälse erscheint jedoch aus derzeitiger Sicht die Anpassung des CCD-Winkels an die Prothesenhalslänge als bevorzugte Variante.

Ein weiterer wesentlicher Aspekt besteht in einer im Querschnitt abgeflachten Ausbildung des Prothesenhalses. Dessen Querschnittsgestalt wird dabei bis zu einem gewissen Grade an den blattartigen Querschnitts des Femur-Verankerungsabschnitts angeglichen; das Spektrum umfaßt mehr oder minder flache Ellipsen, in den Eckbereichen abgerundete Rechtecke, Kombinationen aus Kreisabschnitten und Geraden o. ä.. Speziell in Verbindung mit der oben erwähnten Variante eines Prothesenschaftes mit einem Versatz im Längsverlauf des Prothesenhalses ist in speziellen Ausführungen eine sich im Längsverlauf des Halses ändernde Querschnittsgestalt von Vorteil. So kann ein im Bereich des medialen Bogens zunächst elliptischer Querschnitt proximal des Versatzabschnittes kreisförmig werden, oder der Querschnitt der Ellipse kann sich ändern. In einer anderen Ausführung kann der Halsquerschnitt distal vom Achsenversatzpunkt annähernd rechteckig sein, während er proximal dieses Punktes quadratisch ist oder eine veränderte Rechteckform aufweist.

In einer speziellen, vorteilhaft an die anatomischen Verhältnisse angepaßten Ausführung hat der Schaftquerschnitt mindestens im proximalen Bereich annähernd eine Trapezform, insbesondere eine symmetrische Trapezform mit zwei gleich langen längeren Seitenkanten, die im Querschnitt die anterior bzw. posterior gelegenen Seitenflächen des Schaftes begrenzen, und zwei unterschiedlich langen kürzeren Seitenkanten, von denen die kürzere der medialen Stimfläche und die längere der lateralen Stirnfläche des Schaftes entspricht.

Weitere vorteilhafte konstruktive Maßnahmen und Alternativen der erfindungsgemäßen Konstruktion sind in den Unteransprüchen sowie den folgenden Ausführungsbeispielen anhand derbeigefügten Zeichnungen näher erläutert. Diese zeigen in
- Fig. 1: eine Seitenansicht einerblattartigen Schaftprothese entsprechend dem Stand der Technik, in die skizzenartig mögliche Lagen verschiedener Gelenkkugeln bei unterschiedlich langen Halsabschnitten mit den entsprechenden Referenzpunkten sowie Abständen zur Schaftlängsachse eingetragen sind,
- Fig. 2: eine schematische Darstellung zur Erläuterung einer ersten Ausführungsform der Erfindung,
- Fig. 3: eine schematische Darstellung zur Erläuterung einer zweiten Ausführungsform, und
- Fig. 4a - 4d: skizzenartige Darstellungen verschiedener Prothesenhalsquerschnitte.

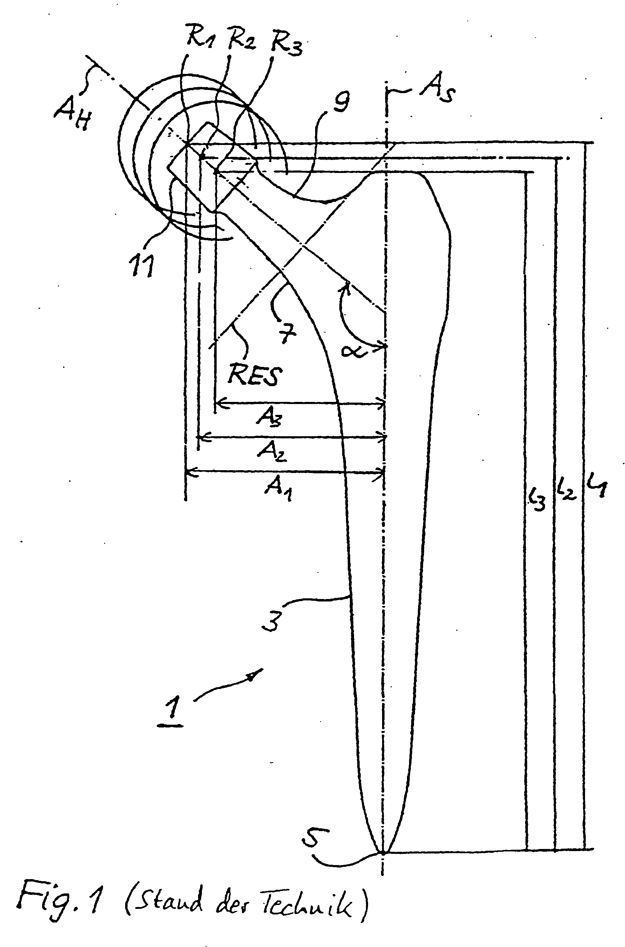

Ein in Fig. 1 dargestellter Prothesenschaft 1 dient zur Verankerung einer Hüftgelenkprothese im Femur. Ausgehend von einem distalen Ende 5, erweitert sich der Prothesenschaft 1 nach proximal allseitig etwa gleichmäßig und geht in einem medialen Bogen 7 in einen Prothesenhals 9 über, der proximal in einem Zapfen 11 zur Aufnahme einer Gelenkkugel endet. Die Lage eines Verankerungsabschnittes 3 des Prothesenschaftes 1 ist durch eine Schaftlängsachse A_{S} beschreibbar, während die Lage des Prothesenhalses durch dessen Längsachse A_{H} beschreibbar ist. Die Schaftlängsachse A_{S} und die Halsachse A_{H} schließen einen Winkel ∝ (üblicherweise bezeichnet als CCD-Winkel) miteinander ein. In Fig. 1 ist weiterhin die bei der Implantation zu beachtende Resektionsebene RES angegeben.

Wie in der Figur skizzenartig angedeutet, können verschiedene Prothesenschäfte der in der Figur gezeigten Art in einem Schaftsystem unterschiedliche Halslängen und damit - bei als konstant vorausgesetztem CCD-winkel ∝ - unterschiedliche Abstände A₁, A₂, A₃ zwischen der Schaftlängsachse A_{S} und einem jeweils zugehörigen Referenzpunkt R₁, R₂, R₃ im Bereich des jeweiligen Zapfens 11 haben. Die Referenzpunkte R₁, R₂, R₃ bezeichnen jeweils den Mittelpunkt einer zugehörigen Gelenkkugel (die in der Figur kein gesondertes Bezugszeichen hat). Auch die Gesamtlängen der einzelnen Prothesen des Systems bzw. die Abstände zwischen dem distalen Ende 5 und dem jeweiligen Referenzpunkt R₁, R₂ bzw. R₃ - in der Figur bezeichnet mit I₁, I₂ und I₃ - sind unterschiedlich. Dies bedingt bei Präparation des Femurs unterschiedliche Raspelmaße und damit, bezogen auf eine größere Patientenanzahl, einen relativ hohen präparativen Aufwand.

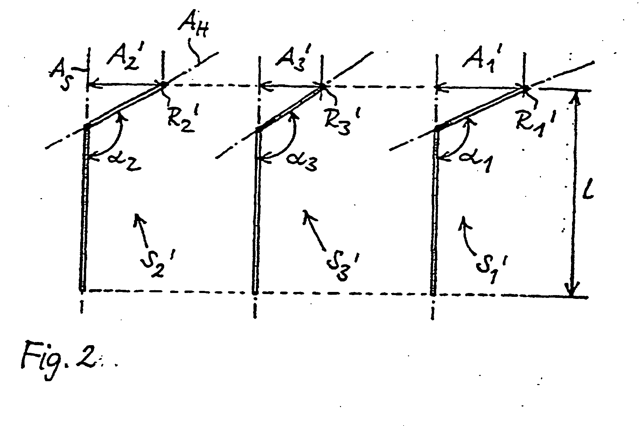

In Fig. 2 ist schematisch eine erste Variante der Ausführung der Erfindung skizziert, die durch das Vorsehen unterschiedlicher CCD-Winkel ∝₁, ∝₂ bzw. ∝₃ bei den Prothesenschäften S₁', S₂' und S₃' eines (hier dreiteiligen) Hüftgelenk-Endoprothesensystems charakterisiert ist. Durch Variation des CCD-Winkels zwischen der Schaftlängsachse A_{S} und der Halsachse A_{H} wird erreicht, daß trotz unterschiedlicher Abstände A₁', A₂', A₃' zwischen der Schaftlängsachse A_{S} und dem jeweiligen Referenzpunkt R₁', R₂', R₃' alle Prothesenschäfte die gleiche Gesamtlänge I haben.

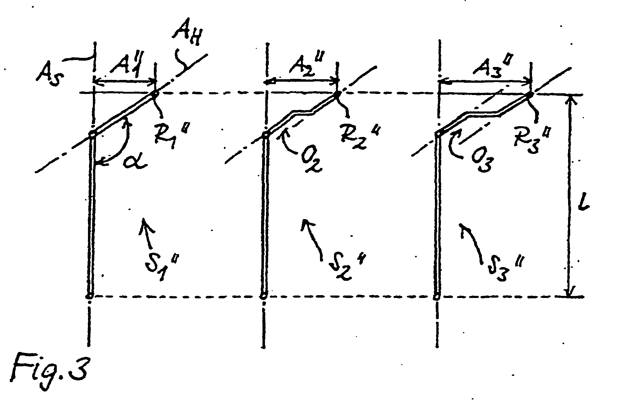

In Fig. 3 ist eine alternative Ausführung der Erfindung anhand von drei jeweils grob schematisch dargestellten Prothesenschäften S₁", S₂" bzw. S₃" dargestellt. Die Besonderheit dieser Ausführung besteht im Vorsehen eines Versatzes O₂ bzw. O₃ im Verlauf des Prothesenhalses bei den Prothesenschäften S₂" und S₃". Durch diesen Versatz (Offset) wird erreicht, daß trotz unterschiedlicher Abstände zwischen den Referenzpunkten R₁", R₂" bzw. R_{3'}' und der Schaftlängsachse A_{S} und einheitlichem Winkel ∝ zwischen dieser und der Halsachse A_{H} eine einheitliche Gesamtlänge I für alle Prothesenschäfte des Systems realisiert werden kann.

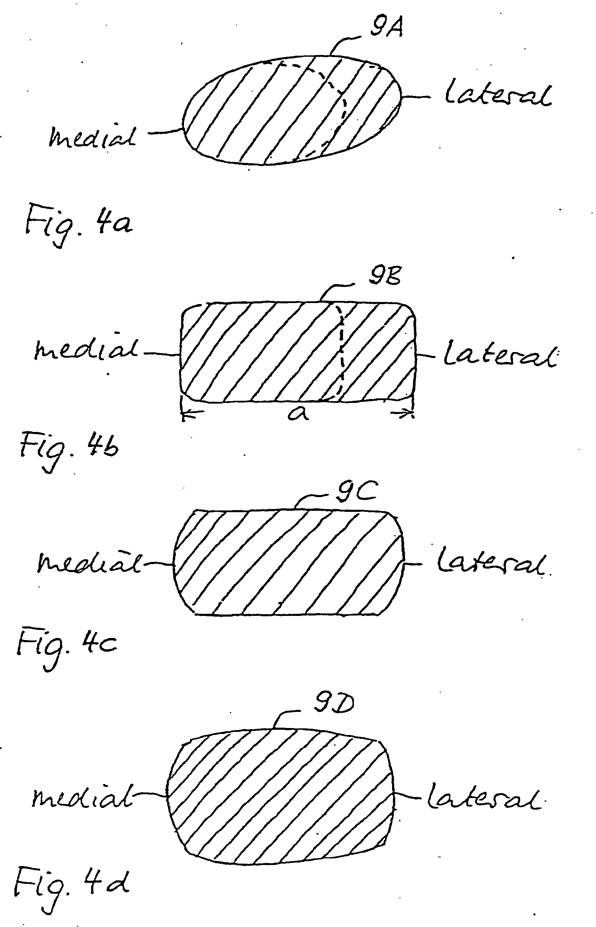

In Fig. 4a bis 4d sind - lediglich als Beispiele für eine Vielzahl möglicher konkreter Querschnittsformen zu verstehen - einige bevorzugte Ausführungen des Prothesenhalses 9.1' bzw. 9.2' eines Hüftgelenk-Prothesenschaftes im Querschnitt gezeigt. Zur Unterscheidung voneinander sind die Prothesenhälse in diesen Figuren mit 9A, 9B, 9C bzw. 9D bezeichnet.

Bei der Ausführung nach Fig. 4a ergibt die anterior-posteriore Abflachung einen elliptischen Querschnitt, wobei die große Achse der Ellipse im wesentlichen in mediat-lateraler Richtung verläuft. Fig. 4b zeigt eine andere Variante, bei der der Prothesenhals den Querschnitt eines in den Eckbereichen abgerundeteten Rechtecks hat. Die längere Rechtecksseite a erstreckt sich hier - analog zur großen Achse der Ellipse nach Fig. 4a - in medial-lateraler Richtung. Bei der in Fig. 4c gezeigten Ausführung sind die anteriore und posteriore Begrenzung der Querschnittsform Geraden, während die mediale und laterale Begrenzungslinie jeweils Kreisbogenabschnitte sind. Der in Fig. 4d gezeigte Prothesenhals 9D hat eine Querschnittsform, bei der alle Begrenzungslinien Kreisbogenabschnitte sind, wobei die die anteriore und posteriore Begrenzung bildenden Kreisbögen einen größeren Radius als die die mediale Begrenzung bildenden Kreisbögen haben.

In Fig.4a und 4b ist jeweils mit einer gestrichelten Linie die Außenkonturdes entsprechenden Prothesenhalses 9A bzw. 9B proximal eines Versatz-Punktes gezeigt, wie er bei Prothesenschäften des in Fig. 3 skizzierten Systems existiert. Bei einersolchen Ausführung variiertalso der Halsquerschnitt in dem Bereich proximal des Versatzabschnittes gegenüber dem Bereich distal dieses Abschnittes, was in vorteilhafter Weise eine einheitliche Außenkontur des Schaftes im Bereich des medialen Bogens trotz eines mehroder weniger großen Offset bei einzelnen Prothesen des Systems möglich machen kann.

Der Querschnitt des Verankerungsabschnitts der Prothesenschäfte kann bei den hier vorgeschlagenen Ausführungen die bei blattartigen Prothesenschäften bekannten Formen haben; besonders vorteilhaft ausführbar ist die vorgeschlagene Lösung aber mit einem im wesentlichen trapezförmigen Schaftquerschnitt, der besonders gut an die anatomischen Gegebenheiten angepaßt ist. Die Gestalt dieses Trapezes ist bevorzugt im wesentlichen symmetrisch, wobei die anteriore und posteriore Seitenkante gleich lang und länger als die laterale und die mediale Seitenkante sind, von denen wiederum die mediale Seitenkante die kürzere ist. In den Kantenbereichen des entsprechenden Verankerungsabschnittes (d. h. an den Ecken des die Querschnittsgestalt bestimmenden Trapezes) sind bevorzugt Fasen bzw. Facetten vorgesehen.

Sämtliche in den Anmetdungsunterlagen offenbarten Merkmale werden als erfindungswesentlich beansprucht, soweit sie einzeln oder in Kombination gegenüber dem Stand der Technik neu sind.

### Bezugszeichenliste

- 1; 1.1', 1.2': Prothesenschaft
- 3; 3.1', 3.2': Verankerungsabschnitt
- 5: distales Ende
- 7: medialer Bogen
- 9, 9A, 9B, 9C, 9D: Prothesenhals
- 11: Zapfen
- A_{S}: Schaftlängsachse
- A_{H}: Halsachse
- A₁, A₂, A₃; A₁',A₂',A₃'; A₁", A₂", A₃": Abstand zwischen Schaftlängsachse und Referenzpunkt
- I, I₁, I₂, I₃: Schaftlänge
- R₁, R₂, R₃; R₁', R₂', R₃'; R₁", R₂", R₃": Referenzpunkt
- RES: Resektionsebene
- S₁', S₂', S₃'; S₁", S₂", S₃ ": Prothesenschaft
- ∝, ∝₁, ∝₂, ∝₃: CCD-Winkel

## Patentansprüche

1. Hüftgelenk-Endoprothesensystem mit einer Mehrzahl von, insbesondere blattartigen, Prothesenschäften (1.1', 1.2'; S₁', S₂', S₃' ;S₁", S₂", S₃") zur Verankerung im Femur, wobei jeder Schaft sich von einem distalen Ende (5) aus nach proximal im wesentlichen allseitig erweitert und einen Verankerungsabschnitt mit einer Schaft-Längsachse (A_{S}) aufweist, welcher medial in einen Bogen (7.1', 7.2') übergeht, der sich in einen Prothesenhals (9.1', 9.2') mit einer Prothesenhalsachse (A) fortsetzt, wobei ein Referenzpunkt-Abstand (A₁', A₂', A₃'; A₁", A₂" ; A₂"') zwischen der Schaftlängsachse und einem Referenzpunkt (R₁', R₂', R₃'; R₁", R₂", R₃") auf der Prothesenhalsachse, der die Lage des Mittelpunktes einer auf den Prothesenhals aufgesetzten Gelenkkugel bezeichnet, bei den Prothesenschäften unterschiedlich ist, bei unverändertem Schnittpunkt zwischen Schaftlängsachse (A_{S}) und Prothesenhalsachse (A_{H})
**dadurch gekennzeichnet, daß** die Länge (l) der Projektion auf die Schaft-Längsachse (A_{S}) der Gesamterstreckung des Prothesenschaftes vom distalen Ende des Verankerungsabschnittes bis zum Referenzpunkt bzw. zum proximalen Ende des Prothesenhalses bei allen Prothesenschäften gleich ist.

2. Hüftgelenk-Endoprothesensystem nach Anspruch 1,
**dadurch gekennzeichnet,**
**daß** bei den Prothesenschäften (S₁', S₂', S₃'; 1.1', 1.2') mit unterschiedlichem Referenzpunkt-Abstand (A₁', A₂', A₃') ein Prothesenhalswinkel (∝₁, ∝₂, ∝₃; ∝'_{1.1} ∝'_{1.2}) zwischen der Schaftlängsachse (A_{S}) und der Prothesenhalsachse (A_{H}) in Abhängigkeit vom Referenzpunkt-Abstand unterschiedlich bestimmt ist.

3. Hüftgelenk-Endoprothesensystem nach Anspruch 1 oder 2,
**dadurch** gekennzelchnet,
daß mindestens bei einem der Prothesenschäfte (S₁", S₂", S₃") mit unterschiedlichen Referenzpunkt-Abstand (A₁", A₂", A₃") im Längsverlauf des Prothesenhalses ein Achsenversatz (O₁, O₂) in Richtung nach distal vorgesehen ist, dessen Betrag in Abhängigkeit vom Referenzpunkt-Abstand bestimmt ist.

4. Hüftgelenk-Endoprothesensystem nach einem der vorangehenden Ansprüche,
**dadurch gekennzeichnet,**
**daß** die maximale Erstreckung des Prothesenhalses (9.1', 9.2'; 9A - 9D) quer zur Prothesenhalsachse (A_{H}) in anterior-posteriorer Richtung mindestens In einem Teilabschnitt seines Längsverlaufes geringer als die maximale Erstreckung in medial-lateraler Richtung ist.

5. Hüftgelenk-Endoprothesensystem nach Anspruch 4,
**dadurch gekennzeichnet,**
**daß** der Querschnitt des Prothesenhalses (9.1', 9.2'; 9A-9D) mindestens in einem Teilabschnitt seiner Erstreckung im wesentlichen elliptisch oder rechteckig ist oder mindestens eine gerade und eine gekrümmte Begrenzungslinie aufweist.

6. Hüftgelenk-Endoprothesensystem nach Anspruch 3 und Anspruch 4 oder 5,
**dadurch gekennzeichnet**,
daB der Querschnitt des Prothesenhalses (9A, 9B) in einem Bereich distal des Achsenversatzpunktes abweichend vom Querschnitt in einem Bereich proximal des Achsenversatzpunktes ausgebildet ist.

7. Hüftgelenk-Endoprothesensystem nach einem der vorangehenden Ansprüche,
**dadurch gekennzeichnet,**
**daß** der Verlauf einer den Verankerungsabschnitt (3.1', 3.3') mit dem Prothesenhals (9.1', 9.2') verbindenden medialen Bogenlinie (7.1', 7.2') bei allen Prothesenschäften (1.1', 1.2') mindestens über den größten Teil ihrer Länge identisch ist.

## Claims

1. A hip joint endoprosthesis system comprising a plurality of, in particular blade-type, prosthesis shafts (1.1', 1.2'; S₁', S₂', S₃'; S₁", S₂", S₃") for anchorage in the femur, wherein each shaft widens from a distal end (5) in a proximal direction substantially on all sides and has an anchoring portion having a longitudinal shaft axis (As) and merging medially into a curve (7.1', 7.2') which continues into a prosthesis neck (9.1', 9.2') having a prosthesis neck axis (A_{H}), wherein a reference point distance (A₁', A₂', A₃'; A₁", A₂"; A₂"') between the longitudinal shaft axis and a reference point (R₁', R₂', R₃'; R₁", R₂", R₃") on the prosthesis neck axis designates the position of the centre point of a ball attached to the prosthesis neck and varies in the prosthesis shafts, **characterised in that**, with an unchanged point of intersection between the longitudinal shaft axis (A_{S}) and the prosthesis neck axis (A_{H}), the length (L) of the projection onto the longitudinal shaft axis (A_{S}) of the overall extent of the prosthesis shaft from the distal end of the anchoring portion to the reference point or the proximal end of the prosthesis neck is the same in all prosthesis shafts.

2. A hip joint endoprosthesis system according to claim 1, **characterised in that**, in the prosthesis shafts (S₁', S₂', S₃'; 1.1', 1.2') with different reference point distances (A₁', A₂', A₃'), a prosthesis neck angle (α₁, α₂, α₃; α'_{1.1}, α'_{1.2}) between the longitudinal shaft axis (A_{S}) and the prosthesis neck axis (A_{H}) is varyingly determined as a function of the reference point distance.

3. A hip joint endoprosthesis system according to claim 1 or 2, **characterised in that**, in at least one of the prosthesis shafts (S₁", S₂", S₃") with different reference point distances (A₁", A₂", A₃"), an axial offset (O₁, O₂) is provided in the longitudinal extent of the prosthesis neck in a distal direction and its size is determined as a function of the reference point distance.

4. A hip joint endoprosthesis system according to any one of the preceding claims, **characterised in that** the maximum extent of the prosthesis neck (9.1', 9.2'; 9A-9D) transversely to the prosthesis neck axis (A_{H}) in the anterior-posterior direction is smaller at least over a portion of its length than the maximum extent in the medial-lateral direction.

5. A hip joint endoprosthesis system according to claim 4, **characterised in that** the cross-section of the prosthesis neck (9.1', 9.2'; 9A-9D) is substantially elliptical or rectangular at least over a portion of its extent or has at least one straight and one curved boundary line.

6. A hip joint endoprosthesis system according to claim 3 and claim 4 or 5, **characterised in that** the cross-section of the prosthesis neck (9A, 9B) in a region distally of the axial offset point is formed differently from the cross-section in a region proximally of the axial offset point.

7. A hip joint endoprosthesis system according to any one of the preceding claims, **characterised in that** the path of a medial curve (7.1', 7.2') connecting the anchoring portion (3.1', 3.3') to the prosthesis neck (9.1', 9.2') is identical in all prosthesis shafts (1.1', 1.2') at least over the greatest part of their length.

## Revendications

1. Système d'endoprothèse de la hanche avec une pluralité de tiges de prothèses, en particulier en forme de feuille (1.1', 1.2' ; S₁', S₂', S₃' ; S₁'', S₂'', S₃''), pour l'ancrage dans le fémur, chaque tige s'élargissant depuis une extrémité distale (5) en direction proximale de façon sensiblement polydireationnelle et une section d'ancrage présentant un axe longitudinal de tige (Aₛ) qui prend une forme d'arc en son milieu (7,1', 7.2'), qui se prolonge par un col de prothèse (9.1', 9.2') avec un axe de col de prothèse (A_{H}), un écartement de point de référence (A₁', A₂', A₃' ; A₁'', A₂'' ; A₂''') entre l'axe longitudinal de la tige et un point de référence (R₁', R₂', R₃' ; R₁'', R₂'', R₃'') sur l'axe du col de la prothèse, qui désigne la position du centre d'une tête d'articulation placée sur le col de la prothèse, étant différent selon les tiges de prothèse, **caractérisé en ce que** lorsqu'est constant le point d'intersection entre l'axe longitudinal de la tige (Aₛ) et l'axe de col de prothèse (A_{H}), la longueur (L) de la projection sur l'axe longitudinal de la tige (Aₛ) de l'étendue complète de la tige de prothèse depuis l'extrémité distale de la section d'ancrage jusqu'à un point de référence, c'est-à-dire jusqu'à l'extrémité proximale du col de la prothèse, est la même pour toutes les tiges de prothèse.

2. Système d'endoprothèse de la hanche selon la revendication 1, **caractérisé en ce qu'**un angle de col de prothèse (α₁, α₂, α₃; α'₁₋₁, α'₁₋₂) est déterminé sur les tiges de prothèse (S₁', S₂', S₃' ; 1.1', 1.2') entre l'axe longitudinal de la tige (Aₛ) et l'axe du col de prothèse (A_{H}) par les différents écartements de point de référence (A'₁, A'₂, A'₃) de manière différente selon chaque écartement de point de référence.

3. Système d'endoprothèse de la hanche selon la revendication 1 ou selon la revendication 2, **caractérisé en ce qu'**est prévu, au moins pour une des tiges de prothèse (S₁'', S₂'', S₃''), avec des écartements de point de référence différents (A₁'', A₂'', A₃'') sur la longueur du col de la prothèse, un décalage d'axe (O₁, O₂) en direction distale, dont l'ampleur est déterminée en fonction de l'écartement de point de référence.

4. Système d'endoprothèse de la hanche selon l'une des revendications précédentes, **caractérisé en ce que** l'étendue maximale en direction antéropostérieure du col de la prothèse (9.1', 9.2' ; 9A-9D) en diagonale par rapport à l'axe du col de la prothèse (A_{H}) est plus étroite que l'étendue maximale en direction médio-latérale au moins sur une section partielle de la longueur.

5. Système d'endoprothèse de la hanche selon la revendication 4, **caractérisé en ce que** la coupe du col de la prothèse (9.1', 9.2' ; 9A-9D), au moins sur une section partielle de son étendue, est généralement elliptique ou rectangulaire ou présente au moins une ligne de séparation droite et une ligne de séparation courbée.

6. Système d'endoprothèse .de la hanche selon la revendication 3 et selon la revendication 4 ou 5, **caractérisé en ce que** la coupe du col de la prothèse (9A, 9B), dans une zone en direction distale du point de décalage de l'axe, est formée de manière différente de cette coupe dans une zone en direction proximale du point de décalage de l'axe.

7. Système d'endoprothèse de la hanche selon l'une des revendications précédentes, **caractérisé en ce que** le tracé de l'une des lignes d'arc médianes (7.1', 7.2') reliant la section d'ancrage (3.1', 3.3') au col de la prothèse (9.1', 9.2') est identique pour toutes les tiges de prothèse (1.1', 1.2') au moins sur la plus grande partie de sa longueur.
